# FASCICULE DE BREVET EUROPEEN

(11) **EP 1 940 770 B1**
(45) Date de publication et mention de la délivrance du brevet: **11.11.2015**
(21) Numéro de dépôt: 06820174.8
(22) Date de dépôt: 10.10.2006
(51) Int. Cl.: C07C 67/343, C07C 51/09, C07C 51/377

(54) **NOUVEAU PROCÉDÉ DE PRÉPARATION D'HYDROXY-ACIDES GRAS INSATURÉS**
NEUES VERFAHREN ZUR HERSTELLUNG UNGESÄTTIGTER HYDROXYFETTSÄUREN
NOVEL METHOD FOR PREPARING UNSATURATED FATTY HYDROXYACIDS

(30) Priorité: 21.10.2005 FR 0510765
(43) Date de publication de la demande: 09.07.2008
(73) Titulaire: Pierre Fabre Dermo-Cosmétique, 92100 Boulogne-Billancourt (FR)
(72) Inventeur: FRISON, Natacha, F-60100 Creil (FR); FOLLEAS, Benoît, F-60300 Senlis (FR); BRAYER, Jean-Louis, F-60440 Nanteuil le Haudouin (FR)
(74) Mandataire: Regimbeau
(86) Numéro de dépôt international: PCT/FR2006/002268
(87) Numéro de publication internationale: WO 2007/045741

(56) Documents cités:
- FR-A- 2 684 988
- JP-A- 63 317 091
- VILLIERAS J ET AL: "La Reaction de Wittig-Horner en milieu heterogene VI. Selectivité de la reaction sur des composés bifonctionnels" TETRAHEDRON LETTERS, ELSEVIER, AMSTERDAM, NL, vol. 26, no. 1, 1985, pages 53-56, XP002198720 ISSN: 0040-4039
- RAGOUSSIS: "Stereoselektive Access to Tetrahydropyranylacetic Acid Derivatives" SYNTHESIS, vol. 1, 1993, pages 84-86, XP001247243 cité dans la demande
- LIST ET AL: "Practical Synthesis of (E)-alpha,beta-Unsaturated Esters from Aldehydes" ADV. SYNTH. CATAL., vol. 347, 2005, pages 1558-1560, XP002390820
- COLONGE ET AL: "Préparation d'homologues du décène-2 ol-10 oique" BULL. SOC.CHIM., 1963, pages 551-553, XP009069614
- DATABASE BEILSTEIN BEILSTEIN INSTITUTE FOR ORGANIC CHEMISTRY, FRANKFURT-MAIN, DE; XP002432342 Database accession no. BRN1867281 -& S.DOLEZAL ET AL: COLLECT.CZECH.CHEM.COMMUN., vol. 31, 1966, pages 3765-3774, XP009083434
- DATABASE BEILSTEIN BEILSTEIN INSTITUTE FOR ORGANIC CHEMISTRY, FRANKFURT-MAIN, DE; XP002432343 Database accession no. BRN239063 -& YOKOI ET AL: NIPPON KAGAKU KAISHI, 1978, pages 1415-1417, XP009083436
- DATABASE BEILSTEIN BEILSTEIN INSTITUTE FOR ORGANIC CHEMISTRY, FRANKFURT-MAIN, DE; XP002432344 Database accession no. BRN1860078 -& J.CHEM.SOC., 1962, pages 5299-5301, XP009083435

## Description

La présente invention a pour objet un nouveau procédé de préparation d'hydroxy-acides gras insaturés. Elle a également pour objet de nouveaux hydroxy-acides gras insaturés, ainsi que leur utilisation, notamment dans le domaine cosmétique.

De nombreux hydroxy-acides gras saturés sont connus et décrits dans la littérature pour leurs propriétés biologiques et plus particulièrement pour leurs propriétés cosmétiques et pharmacologiques. Par exemple, le principal constituant lipidique de la gelée royale des abeilles est un hydroxy-acide gras insaturé, à savoir l'acide hydroxy-10-décène-2(trans)oïque (Edward E. Smissman et al., 1964, JOC, 29, 3517-3520).

Différents documents de l'état de la technique décrivent des procédés de préparation des hydroxy-acides gras insaturés et de leurs esters (Lee et al., 1993, J. Org. Chem., 58, 2918-2919 ; Hurd et Saunders, 1952, J. Am. Chem. Soc., 74, 5324-5328 ; Krishnamurthy et al., 1989, Indian J. Chem. Sect. A, 28, 288-291 ; Plettner et al., 1995, J. Chem. Ecol., 21, 1017-1030).

Les procédés déjà connus dans l'état de la technique présentent une étape d'oxydation durant laquelle des sels métalliques comme les sels de chrome ou de manganèse sont employés. Or, l'utilisation de sels métalliques présente un certain nombre d'inconvénients. D'une part, au niveau des produits obtenus par lesdits procédés, ces derniers peuvent être contaminés par les sels métalliques et donc leur application cosmétique et/ou pharmacologique est limitée du fait de cette contamination. D'autre part, l'utilisation de sels métalliques entraîne une contamination de l'environnement des industries dans lesquelles la synthèse est effectuée.

D'autres procédés déjà connus dans l'état de la technique montrent qu'une homologation de deux carbones, soit par une réaction de Doebner Knoevenagel, soit par une réaction de Wittig, sur un lactol (lactone partiellement réduite) de petite taille conduit de façon minoritaire à des hydroxy-acides insaturés et de façon majoritaire à des acides tétrahydropyranylacétique ou tétrahyrofuranylacétique (Ragoussis et al., 1993, Synthesis, 1, 84-86). Ainsi, de tels procédés ne permettent pas d'obtenir des hydroxy-acides gras insaturés dans des quantités satisfaisantes.

La présente invention a pour but de fournir un nouveau procédé de préparation d'hydroxy-acides gras insaturés, dans d'excellentes conditions à la fois en termes de rendement mais aussi de qualité sans trace de contamination notamment par des produits cyclisés, ledit procédé pouvant être transposé au niveau industriel.

La présente invention a pour but de fournir une méthode de synthèse d'hydroxy-acides gras insaturés plus rapide et présentant de meilleurs rendements que les procédés actuellement utilisés, le rendement du procédé de l'invention étant augmenté de 100 à 200% par rapport aux procédés de l'état de la technique.

La présente invention a également pour but de fournir un procédé simplifié par rapport aux procédés de l'état de la technique, et ce notamment en raison d'un nombre réduit d'étapes.

La présente invention a également pour but de fournir un procédé qui ne comprend pas d'étape d'oxydation et qui ne comprend pas l'utilisation de sels de métaux lourds, afin d'éviter les problèmes de contamination avec ces sels de métaux lourds.

Enfin, la présente invention a pour but de fournir un procédé qui permet d'obtenir toute une gamme de produits, dont certains sont obtenus à partir de produits intermédiaires non disponibles dans le commerce, mais dont la synthèse est possible.

La présente invention concerne un procédé de préparation d'un composé de formule (I) suivante : 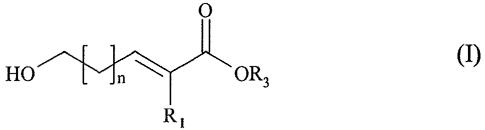 dans laquelle :
- R₃ représente H ou un groupe alkyle R₄, linéaire ou ramifié, comprenant de 1 à 6 atomes de carbone,
- R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
- n est égal à 7, 8, 9, 10, 11, 12, 13 ou 14,
ledit procédé de préparation comprenant :
- la mise en oeuvre de la réaction de Wittig-Horner par la réaction d'un phosphonate de formule (III) suivante : 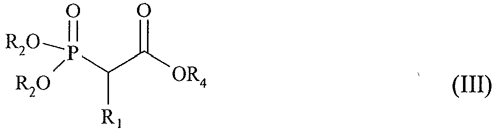
dans laquelle :
- R₁ est tel que défini ci-dessus,
- R₂ représente un groupe alkyle, linéaire ou ramifié, comprenant de 1 à 6 atomes de carbone, et est de préférence un groupe méthyle ou éthyle, lesdits groupes R₂ pouvant former un cycle avec les atomes d'oxygène des groupes OR₂ et l'atome de phosphore du groupe P=O, et
- R₄ représente un groupe alkyle, linéaire ou ramifié, comprenant de 1 à 6 atomes de carbone, et est de préférence un groupe éthyle,
   sur un lactol de formule (II) suivante : 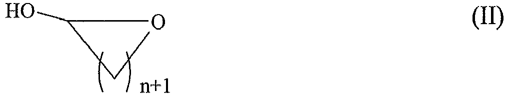 n étant tel que défini ci-dessus,
   afin d'obtenir un hydroxy-ester de formule (IV) suivante : 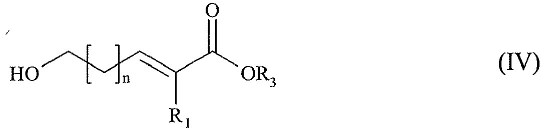 dans laquelle n, R₃ et R₁ sont tels que définis ci-dessus,
- et, éventuellement, lorsque R₃ représente un groupe R₄ tel que défini ci-dessus, une réaction de saponification de l'hydroxy-ester de formule (IV) susmentionnée, pour obtenir un hydroxy-acide de formule (I-1) suivante : 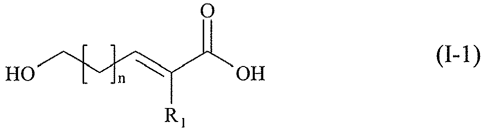 dans laquelle n et R₁ sont tels que définis ci-dessus.

Les composés de formule (I) susmentionnée englobent les composés de formule (IV) et les composés de formule (I-1). Ainsi, les composés de formule (IV) telle que définie ci-dessus correspondent à des composés de formule (I) dans laquelle R₃ représente un atome d'hydrogène ou un groupe alkyle R₄ tel que défini ci-dessus, et les composés de formule (I-1) telle que définie ci-dessus correspondent à des composés de formule (I) dans laquelle R₃ représente un atome d'hydrogène.

La réaction de Wittig- Horner est une réaction décrite dans le document Modem Synthetic Reaction, Second édition, Herbet O. House, Wittig Horner reaction p.682-709, et toute condition expérimentale décrite dans l'état de la technique peut être utilisée dans le cadre de la présente invention. A titre d'exemple, la réaction de Wittig-Horner peut être effectuée en présence de triéthylphosphonoacétate et de carbonate de potassium, en milieu aqueux. Dans ce dernier cas l'acide est obtenu directement sans hydrolyse supplémentaire (Références : Introduction of a carbon chain or an aromatic ring vol II, Jean Mathieu and Jean Weill-Raynal, Georg Thieme Publishers, 1975, Chapitre : "alkylidenation of methylene groups by means of aldehydes and ketones, p. 513-521).

La présente invention concerne également un procédé de préparation d'un composé de formule (IV) suivante : 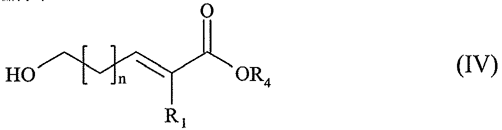 dans laquelle :
- R₄ représente un groupe alkyle, linéaire ou ramifié, comprenant de 1 à 6 atomes de carbone,
- R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
- n est égal à 7, 8, 9, 10,11, 12, 13 ou 14,
ledit procédé de préparation comprenant :
- la mise en oeuvre de la réaction de Wittig-Horner par la réaction d'un phosphonate de formule (III) suivante : 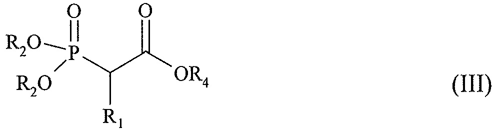
dans laquelle :
- R₁ est tel que défini ci-dessus,
- R₂ représente un groupe alkyle, linéaire ou ramifié, comprenant de 1 à 6 atomes de carbone, et est de préférence un groupe éthyle ou méthyle, lesdits groupes R₂ pouvant former un cycle avec les atomes d'oxygène des groupes OR₂ et l'atome de phosphore du groupe P=O, et
- R₄ représente un groupe alkyle, linéaire ou ramifié, comprenant de 1 à 6 atomes de carbone, et est de préférence un groupe éthyle,
   sur un lactol de formule (II) suivante : 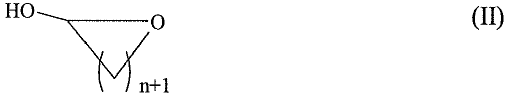 n étant tel que défini ci-dessus,
   afin d'obtenir un hydroxy-ester de formule (IV) suivante : 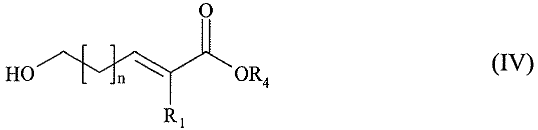 dans laquelle n, R₁ et R₄ sont tels que définis ci-dessus.

La présente invention concerne également un procédé de préparation d'un composé de formule (I-1) suivante : 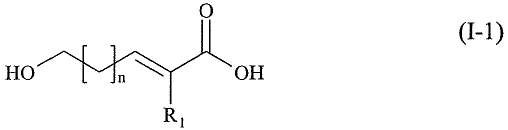 dans laquelle :
- R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
- n est égal à 7, 8, 9, 10,11, 12, 13 ou 14,
ledit procédé de préparation comprenant :
- la mise en oeuvre de la réaction de Wittig-Horner par la réaction d'un phosphonate de formule (III) suivante : 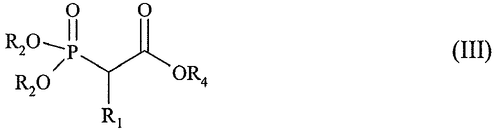
dans laquelle :
- R₁ est tel que défini ci-dessus,
- R₂ représente un groupe alkyle, linéaire ou ramifié, comprenant de 1 à 6 atomes de carbone, et est de préférence un groupe éthyle ou méthyle, lesdits groupes R₂ pouvant former un cycle avec les atomes d'oxygène des groupes OR₂ et l'atome de phosphore du groupe P=O, et
- R₄ représente un groupe alkyle, linéaire ou ramifié, comprenant de 1 à 6 atomes de carbone, et est de préférence un groupe éthyle,
   sur un lactol de formule (II) suivante : 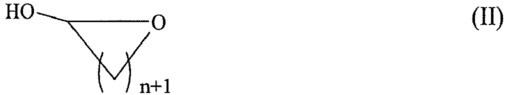 n étant tel que défini ci-dessus,
   afin d'obtenir un hydroxy-ester de formule (IV) suivante : 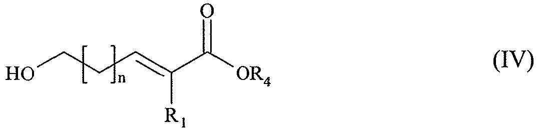 dans laquelle n, R₁ et R₄ sont tels que définis ci-dessus,
- et une réaction de saponification de l'hydroxy-ester de formule (IV) susmentionnée, pour obtenir un hydroxy-acide de formule (I-1) suivante : 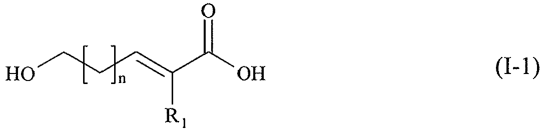 dans laquelle n et R₁ sont tels que définis ci-dessus.

Selon un mode de réalisation préféré, le procédé de l'invention tel que défini ci-dessus est caractérisé en ce que le lactol de formule (II) est obtenu par la réduction d'une lactone de formule (V) suivante :

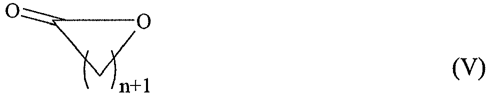

n étant tel que défini ci-dessus.

L'étape de réduction partielle de la lactone (V) en lactol de formule (II) est effectuée en présence d'un agent réducteur tel que le diisobutyl aluminium hydrure. En fin de réaction, les sels d'aluminium sont éliminés par formation d'un complexe soluble dans l'eau en présence de sels de Rozen ou sels de Rochelle (Reagents for organic synthesis, vol. I, Louis Fieser and Mary Fieser, p. 36).

De façon avantageuse, les agents réducteurs sont choisis parmi le Dibal H (Posner Gary H et al., 1991, JOC, 56(25), 6981-6987), le Red Al, le OMH1, le tri(terbutoxy) Aluminium lithium hydrure (Mili Shokyo et al., 1990, J. Chem. Soc. Perkin Trans, 1(4), 1228-1229) ou tout autre hydrure d'aluminium dont la réactivité aura été modérée par l'introduction de différents groupements.

Selon un autre mode de réalisation préféré, le procédé de l'invention tel que défini ci-dessus est caractérisé en ce que la lactone de formule (V) est obtenue à partir d'une cétone de formule (VI) suivante, par la mise en oeuvre de la réaction de Baeyer-Villiger :

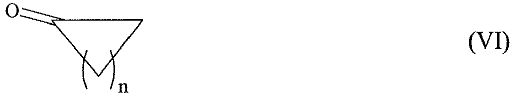

n étant tel que défini ci-dessus.

La réaction de Baeyer-Villiger est notamment décrite dans les articles suivants : Friess SL, 1949, JOC, 71, 2571-2575 ; Kruizinga Wim H. et al., 1981, JACS, 103(17), 5183-5189.

De nombreuses techniques permettant une oxydation d'une cétone (VI) en lactone (V) sont connues dans l'état de la technique et sont utilisables par l'homme du métier à cette étape. Cette oxydation nécessite l'utilisation d'un solvant qui peut notamment être le chlorure de méthylène, le dichloro-1,2-éthane ou tout autre solvant chloré inerte. On peut citer les conditions expérimentales d'oxydation utilisant l'acide 3-chloro perbenzoïque, décrites dans Ishihara et al., 1996, J. Org. Chem., 61, 4560-4567.

La présente invention concerne également un procédé de préparation d'un hydroxy-acide gras insaturé de formule (I-1) suivante : 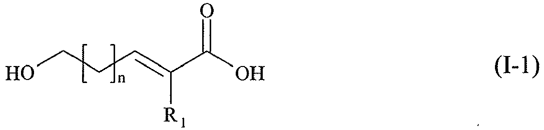 dans laquelle :
- R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
- n est égal à 7, 8, 9, 10,11, 12, 13 ou 14,
ledit procédé de préparation comprenant les étapes suivantes :
- la réduction d'une lactone de formule (V) suivante : 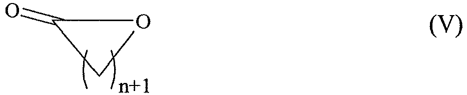 n étant tel que défini ci-dessus,
   afin d'obtenir un lactol de formule (11) suivante : 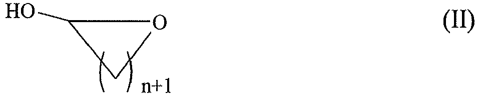 n étant tel que défini ci-dessus,
- la mise en oeuvre de la réaction de Wittig-Horner par la réaction d'un phosphonate de formule (III) suivante : 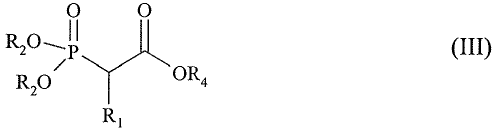 dans laquelle :
- R₁ est tel que défini ci-dessus,
- R₂ représente un groupe alkyle, linéaire ou ramifié, comprenant de 1 à 6 atomes de carbone, et est de préférence un groupe éthyle ou méthyle, lesdits groupes R₂ pouvant former un cycle avec les atomes d'oxygène des groupes OR₂ et l'atome de phosphore du groupe P=O, et
- R₄ représente un groupe alkyle, linéaire ou ramifié, comprenant de 1 à 6 atomes de carbone, et est de préférence un groupe éthyle,
   sur le lactol de formule (II) susmentionnée,
   afin d'obtenir un hydroxy-ester de formule (IV) suivante : 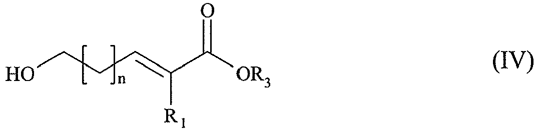 dans laquelle n, R₁ et R₃ sont tels que définis ci-dessus,
- et, lorsque R₃ représente un groupe R₄ tel que défini ci-dessus, une réaction de saponification de l'hydroxy-ester de formule (IV) susmentionnée, pour obtenir un hydroxy-acide de formule (I-1) susmentionnée.

Pour la réaction de Wittig-Horner, la température de travail est de 40°C : cette réaction n'a pas besoin d'un grand apport énergetique . Cette réaction est effectuée en milieu basique avec un agent de déprotonation. Par ailleurs, la réaction de Wittig-Horner nécessite la présence d'une amine secondaire pour l'étape de décarboxylation.

La présente invention concerne également les composés tels qu'obtenus par le procédé tel que défini ci-dessus.

La présente invention concerne également un composé de formule (I-3) suivante : 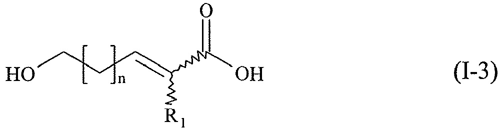 dans laquelle :
- R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
- n est égal à 7, 8, 9, 10, 11, 12, 13 ou 14,
ledit composé étant sous la forme de stéréoisomères Z ou E, ou sous la forme d'un mélange de ces différentes formes.

Les composés de formule (I-3) présentent la particularité d'être moins métabolisables et moins lipophiles que les composés de formule (I-2) suivante:

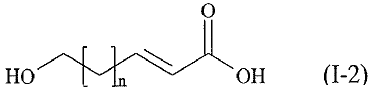

La présente invention concerne une composition cosmétique comprenant un composé tel que défini ci-dessus, répondant à la formule (I-3) telle que définie ci-dessus, en association avec un véhicule approprié.

### Exemple 1 - Mode opératoire pour la synthèse du DHA (exemple comparatif) Obtention du composé de formule suivante avec R₁ = H et n = 6:

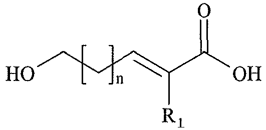

### 1. Préparation de l'Oxonan-2-one

43,5g (345 mmol) de cyclooctanone (composé de formule (VI) avec n = 6)(ACROS) sont mis en solution dans 430 ml de dichloroéthane. 170 g (985 mmol) d'acide *meta*-chloroperbenzoïque sont ensuite ajoutés. Le milieu est chauffé à 80°C pendant 48 heures. A température ambiante, 400 ml d'une solution saturée Na₂S₂O₅ et NaHCO₃ (1/1 v/v) sont ajoutés. Le milieu est agité vigoureusement pendant 18 heures. La phase organique est séparée et mise en présence de KI et H₂O pendant 6 heures. La phase organique est séparée et lavée par une solution saturée de Na₂S₂O₃, par une solution saturée de NaCl, puis est séchée sur MgSO₄, filtrée et concentrée sous vide pour donner un brut de 36 g.

La lactone est purifiée par empâtage dans le pentane (60 ml), puis par filtration du précipité acide *meta*-chlorobenzoïque formé à froid, m=26,6g (54%).

La lactone obtenue est un composé de formule (V) avec n = 6 :

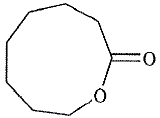

### Caractérisation :

**CCM :** Rf= 0,3 (heptane / acétate d'éthyle 7/3)
**¹H RMN** (300 MHz, CDCl₃) : δ 1,42-1,49 (m, 4H) ; 1,65-1,75 (m, 6H) ; 2,30 (t, *J=* 5,4 Hz, 2H) ; 4,30 (t, *J=* 5,7 Hz, 2H).

### 2. Etape de réduction partielle en lactol

26,6 g (187,2 mmol) de lactone obtenue à l'étape précédente sont dilués dans 210 ml de toluène, sous atmosphère d'azote. Le milieu est refroidi à -78°C et 156,4 ml (189,1 mmol) de Dibal-H (ACROS) en solution à 20% dans le toluène sont additionnés goutte à goutte, en maintenant la température à -78°C. Le mélange est agité pendant 2 heures à -78°C. 200 ml d'une solution saturée de sels de Rozen (sels de tartrate double ; ACROS) sont ajoutés à -78°C. Après 18 heures d'agitation vigoureuse à température ambiante, le mélange biphasique est filtré sur célite, puis extrait à l'acétate d'éthyle. Les phases organiques sont lavées avec une solution saturée de NaCl, séchées sur MgSO₄, filtrées et concentrées sous vide pour donner un brut de 26 g (dont 25% de dérivé diol, soit un rendement estimé à 72%). Le lactol, en équilibre forme ouverte, forme cyclique, est utilisé ainsi, sans purification supplémentaire.

Le lactol obtenu est un composé de formule (II) avec n = 6 :

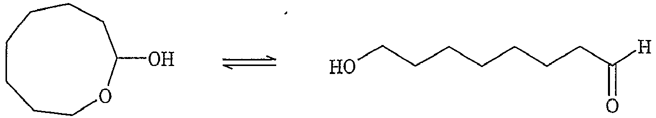

### Caractérisation :

**CCM :** Rf = 0,4 (heptane / acétate d'éthyle 6/4)
**¹H RMN** (300MHz, CDCl₃) : δ 1,34-1,68 (m, 10H) ; 2,45 (t, *J* = 5,4 Hz, 2H) ;
3,66 (t, *J=* 6,6 Hz, 2H) ; 9,78 (t, *J=* 1,8 Hz, 1H).

### 3. Réaction de Wittig-Horner

19 g (131,8 mmol) de lactol obtenu à l'étape précédente sont dilués dans 250 ml d'éthanol. 31,4 ml (158,1 mmol) de triéthylphosphonacétate (composé de formule (III) avec R₂ = R₄ = Et et R₁ = H) sont additionnés au milieu en présence de 27,3 g (197,5 mmol) de carbonate de potassium. Le milieu réactionnel est chauffé à 40°C pendant 18 heures. A température ambiante, le milieu est hydrolysé par 200 ml d'eau distillée et extrait avec de l'acétate d'éthyle. Les phases organiques sont lavées par une solution saturée de NaCl, séchées sur MgSO₄, filtrées et concentrées sous vide pour donner un produit brut de 20 g.

L'ester obtenu est purifié par chromatographie avec une élution heptane / acétate d'éthyle 7/3 : 15 g de produit sont obtenus (53% de rendement).

L'ester obtenu est un composé de formule (IV) avec n = 6, R₁ = H et R₄ = Et :

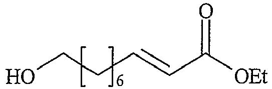

### Caractérisation :

**CCM :** Rf= 0,4 (heptane / acétate d'éthyle 7/3)
**¹H RMN** (300MHz, CDCl₃) : δ 1,24-1,38 (m, 9H) ; 1,43-1,50 (m, 2H) ; 1,51-1,57 (m, 2H) ; 2,15-2,21 (q, 2H) ; 3,60-3,64 (t, 2H) ; 4,14-4,20 (t, 2H) ; 5,77-5,82 (d, *J=* 15,6 Hz, 1H) ; 6,91-6,98 (dt, J = 15,6 Hz, 1H).

### 4. Réaction de saponification

0,60 g (2,81 mmol) d'hydroxy-ester obtenu à l'étape précédente sont solubilisés dans 10 volumes de tétrahydrofurane. Lentement, 3,4 ml (6,75 mmol) d'une solution de soude 2M sont additionnés. Le milieu est chauffé à 65°C pendant 3 heures. Une fois la réaction terminée, le milieu est hydrolysé par l'ajout d'une solution d'acide chlorhydrique 3M, jusqu'à l'obtention d'un pH = 2. Le mélange est concentré à sec et la phase aqueuse est alors extraite par de l'acétate d'éthyle. Les phases organiques sont lavées par une solution saturée de NaCl, séchées sur MgSO₄, filtrées et concentrées sous vide pour donner un produit brut de 0,6 g.

L'hydroxy-acide insaturé attendu est obtenu, sous forme d'un solide blanc, par recristallisation dans l'acétonitrile à froid, m = 0,37g (71%).

L'hydroxy-acide obtenu est un composé de formule (I-2) avec n = 6 :

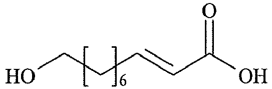

### Caractérisation :

**CCM :** Rf = 0,1 (heptane / acétate d'éthyle 6/4)
**¹H RMN** (300 MHz, CDCl₃) : δ 1,33-1,37 (m, 6H) ; 1,45-1,49 (m, 2H) ; 1,55-1,58 (m, 2H) ; 2,20-2,25 (q, 2H) ; 3,62-3,66 (t, 2H) ; 5,79-5,84 (d, *J*= 15,6 Hz, 1H) ; 7,03-7,10 (dt, *J =* 15,6 Hz, 1H)
**Spectroscopie de Masse :** [M-Na]⁺ 209 (calculé 186)
**Point de fusion :** 62,5°C ± 1°C

### Exemple 2 - Mode opératoire pour la synthèse de l'acide 10-Hydroxy-dec-2-fluoro-2-énoïque

### Obtention d'un composé de formule (I-3) avec R₁ = F et n = 6

Seule l'étape 3 est modifiée (réaction de Wittig-Horner) : à partir du lactol (obtenu à l'étape 2 de l'exemple 1)(0,89 g ; 7,7 mmol), la réaction de Wittig-Horner est conduite en présence de diéthylphosphonofluoroacétate de méthyle (composé de formule (III) avec R₂ = Et, R₁ = F et R₄ = Me)(2,1 g ; 9,2 mmol) et de carbonate de potassium (1,6 g ; 11,5 mmol) dans l'éthanol (9 ml) à 40°C. L'étape 4 (saponification) est conduite selon le protocole précédent. Le produit obtenu après recristallisation, est sous forme d'un mélange *cis*/*trans.*

L'hydroxy-acide obtenu est un composé de formule (I-3) avec R₁ = F et n = 6 :

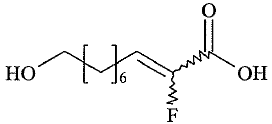

### Caractérisation :

**CCM :** Rf = 0,1 (heptane / acétate d'éthyle 6/4)
**¹H RMN** (300MHz, CDCl₃) : δ 1,36-1,62 (m, 20H) ; 2,27-2,30 (m, 2H, forme *cis) ;* 2,50-2,58 (m, 2H, forme *trans*) ; 3,69 (m, 4H) ; 6,05 (dt, *J =* 21,6 Hz, 1H, forme *trans*) et 6,25 (dt, *J=* 40,8 Hz, 1H, forme *cis).*
**Spectroscopie de Masse :** [M-Na]⁺ 227 (calculé 204)

### Exemple 3 - Mode opératoire pour la synthèse de l'acide 9-hydroxy-nona-2t-énoïque (exemple comparatif)

Le protocole de synthèse de l'exemple 1 est appliqué au cycloheptanone (Aldrich) (composé de formule (VI) avec n = 5) pour conduire à l'acide 9-hydroxy-nona-2*t-*énoïque, qui est un composé de formule (I-2) avec n = 5 :

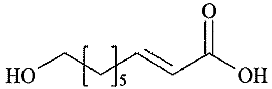

### Caractérisation :

**CCM :** Rf = 0,1 (heptane / acétate d'éthyle 6/4)
**¹H RMN** (300 MHz, CDCl₃) : δ 1,36-1,61 (m, 8H) ; 2,22-2,27 (m, 2H) ; 3,67 (t, *J=* 6,3 Hz, 2H) ; 5,84 (dt, *J =* 15,6 Hz, 1H) ; 7,09 (dt, *J=* 15,6 Hz, 1H).
**Spectroscopie** de **Masse :** [M-Na]⁺ 195 (calculé 172)

### Exemple 4 - Mode opératoire pour la synthèse de l'acide 11-hydroxy-undeca-2t-énoïque (exemple comparatif)

Le protocole de synthèse de l'exemple 1 est appliqué au cyclononanone (Aldrich) (composé de formule (VI) avec n = 7) pour conduire à l'acide 11-hydroxy-undeca-2*t-*énoïque, qui est un composé de formule (I-2) avec n = 7 :

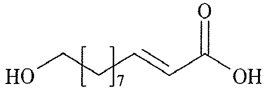

### Caractérisation :

**CCM :** Rf= 0,1 (heptane / acétate d'éthyle 6/4)
**¹H RMN** (300 MHz, CDCl₃) : δ 1,33-1,61 (m, 12H) ; 2,20-2,28 (m, 2H) ; 3,66 (t, *J=* 6,0 Hz, 2H) ; 5,84 (dt, *J=* 15,9 Hz, 1H) ; 7,09 (dt, *J* = 15,9 Hz, 1H).
**Spectroscopie de Masse :** [M-Na]⁺ 223 (calculé 200)

### Exemple 5 - Mode opératoire pour la synthèse de l'acide 12-hydroxy-dodéca-2t-énoïque (exemple comparatif)

Le protocole de synthèse de l'exemple 1 est appliqué au cyclodécanone (Aldrich) (composé de formule (VI) avec n = 8) pour conduire à l'acide 12-hydroxy-dodéca-2*t-*énoïque, qui est un composé de formule (I-2) avec n = 8 :

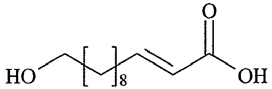

### Caractérisation :

**CCM :** Rf= 0,1 (heptane / acétate d'éthyle 6/4)
**¹H RMN** (300 MHz, CDCl₃) : δ 1,35-1,56 (m, 14H) ; 2,20-2,27 (m, 2H) ; 3,55 (t, *J=* 6,6 Hz, 2H) ; 5,80 (dt, *J=* 15,6 Hz, 1H) ; 6,96 (dt, *J=* 15,6 Hz, 1H).
**Spectroscopie de Masse :** [M-Na]⁺ 237 (calculé 214)

### Exemple 6 - Mode opératoire pour la synthèse de l'acide 12-hydroxy-dodéca-2-fluoro-2t-enoïque : (R1=F, n=8)

Le protocole de synthèse de l'exemple 2 est appliqué au cyclodécanone (Aldrich) (composé de formule (VI) avec n = 8) pour conduire, sous forme d'un mélange *cis*/*trans,* à l'acide 12-hydroxy-dodéca-2-fluoro-2*t*-énoïque, qui est un composé de formule (I-3) avec R₁ = F et n = 8 :

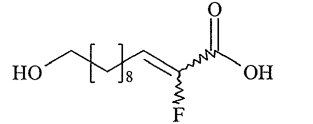

### Caractérisation :

**CCM :** Rf = 0,1 (heptane / acétate d'éthyle 6/4)
**¹H RMN** (300 MHz, CDCl₃): δ 1,34-1,56 (m, 14H) ; 2,24-2,27 (m, 2H forme *cis*) ou 2,49-2,54 (m, 2H structure *trans*) ; 3,55 (t, *J* = 6,6 Hz, 2H) ; 5,97 (dt, *J =* 21,9 Hz, 1 H structure *trans*) ou 6,10 (dt, *J =* 55,2 Hz, 1 H, forme *cis*) **Spectroscopie de Masse :** [M-Na]⁺ 255 (calculé 232)

### Exemple 7 - Mode opératoire pour la synthèse de l'acide 13-hydroxy-tridéc-2t-énoïque (exemple comparatif)

Le protocole de synthèse de l'exemple 1 est appliqué à l'oxacyclododécan-2-one (Aldrich)(composé de formule (V) avec n = 9) pour conduire à l'acide 13-hydroxy-tridec-2*t*-enoïque, qui est un composé de formule (I-2) avec n = 9 :

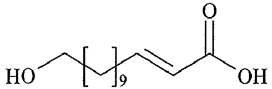

### Caractérisation :

**CCM :** Rf = 0,1 (heptane / acétate d'éthyle 6/4)
**¹H RMN** (300 MHz, CDCl₃) : δ 1,27-1,63 (m, 16H) ; 2,21-2,28 (m, 2H) ; 3,64 (t, *J =* 3,6 Hz, 2H) ; 5,84 (dt, *J* = 15,6 Hz, 1H) ; 7,09 (dt, *J =* 15,6 Hz, 1H) **Spectroscopie de Masse :** [M-Na]⁺ 251 (calculé 228)

### Exemple 8 - Mode opératoire pour la synthèse de l'acide 14-hydroxy-tétradéc-2t-énoïque (exemple comparatif)

Le protocole de synthèse de l'exemple 1 est appliqué à l'oxacyclotridécan-2-one (Aldrich)_(composé de formule (V) avec n = 10) pour conduire à l'acide 14-hydroxy-tétradéc-2*t*-enoïque, qui est un composé de formule (1-2) avec n = 10 :

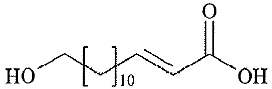

### Caractérisation :

**CCM :** Rf = 0,1 (heptane / acétate d'éthyle 6/4)
**¹H RMN** (300 MHz, CDCl₃) : δ 1,29-1,35 (m, 14H) ; 1,46-1,61 (m, 4H) ; 2,21-2,29 (m, 2H) ; 3,66 (t, *J* = 6,6 Hz, 2H) ; 5,84 (dt, *J* = 15,6 Hz, 1H) ; 7,09 (dt, *J=* 15,6 Hz, 1H).
**Spectroscopie de Masse :** [M-Na]⁺ 265 (calculé 242)

### Exemple 9 - Mode opératoire pour la synthèse de l'acide 14-hydroxy-tétradéc-2-fluoro-2-énoïque (exemple comparatif)

Le protocole de synthèse de l'exemple 2 est appliqué à l'oxacyclotridécan-2-one (Aldrich)_(composé de formule (V) avec n = 10) pour conduire, sous forme d'un mélange *cis*/*trans,* à l'acide 14-hydroxy-tétradéc-2-fluoro-2-énoïque, qui est un composé de formule (I-3) avec R₁ = F et n = 10 :

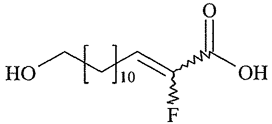

### Caractérisation :

**CCM :** Rf= 0,1 (heptane / acétate d'éthyle 6/4)
**¹H RMN** (300MHz, CDCl₃) : δ 1,29 (m, 28H) ; 1,42-1,62 (m, 8H) ; 2,27-2,31 (m, 2H, forme *cis) ;* 2,51-2,56 (m, 2H, forme *trans*) ; 3,68 (t, *J=* 6,6 Hz, 2H) ; 3,70 (t, *J =* 6,6 Hz, 2H) ; 6,04 (dt, *J =* 21,3 Hz, 1 H, forme *trans*) ; 6,27 (dt, *J =* 33,0 Hz, 1H, forme *cis).*
**Spectroscopie de Masse :** [M-Na]⁺ 283 (calculé 260)

### Exemple 10 - Mode opératoire pour la synthèse de l'acide 17-hydroxy-heptadéc-2t-énoïque (exemple comparatif)

Le protocole de synthèse de l'exemple 1 est appliqué au cyclopentadécanolide (Lancaster)_(composé de formule (V) avec n = 13) pour conduire à l'acide 17-hydroxy-heptadéc-2*t*-énoïque, qui est un composé de formule (I-2) avec n = 13 :

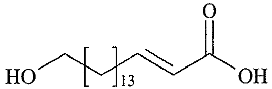

### Caractérisation :

**CCM :** Rf = 0,1 (heptane / acétate d'éthyle 6/4)
**¹H RMN** (300 MHz, CDCl₃): δ 1,27-1,32 (m, 20H) ; 1,46-1,61 (m, 4H) ; 2,20-2,29 (m, 2H) ; 3,67 (t, *J* = 6,6 Hz, 2H) ; 5,84 (dt, *J* = 15,6 Hz, 1H) ; 7,08 (dt, *J=* 15,6 Hz, 1H)
**Spectroscopie de Masse :** [M-Na]⁺ 307 (calculé 284)

### Exemple 11 - Mode opératoire pour la synthèse de l'acide 17-hydroxy-heptadéc-2-fluoro-2-énoïque

Le protocole de synthèse de l'exemple 2 est appliqué au cyclopentadécanolide (Lancaster)(composé de formule (V) avec n = 13) pour conduire, sous forme d'un mélange *cis*/*trans,* à l'acide 17-hydroxy-heptadéc-2-fluoro-2-énoïque, qui est un composé de formule (I-3) avec R₁ = F et n = 13 :

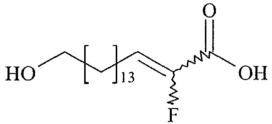

### Caractérisation :

**CCM :** Rf = 0,1 (heptane / acétate d'éthyle 6/4)
**¹H RMN** (300 MHz, CDCl₃) : δ 1,27 (m, 40H) ; 1,45-1,62 (m, 8H) ; 2,24-2,32 (m, 2H, forme *cis) ;* 2,50-2,58 (m, 2H, forme *trans*) ; 3,69 (t, *J*=6,6Hz, 4H) ; 6,05 (dt, *J*= 21,6 Hz, 1H, forme *trans*) et 6,26 (dt, *J* = 40,8 Hz, 1H, forme *cis*) **Spectroscopie de Masse :** [M-H]⁻ 301 (calculé 302)
**Point de fusion :** 77°C ± 1°C

### Exemple 12 - Mode opératoire pour la synthèse de l'acide 18-hydroxy-octadéc-2t-énoïque (exemple comparatif)

Le protocole de synthèse de l'exemple 1 est appliqué au cyclohexadécanolide (Lancaster)(composé de formule (V) avec n = 14) pour conduire à l'acide 18-hydroxy-octadéc-2*t*-énoïque (rendement = 72%), qui est un composé de formule (I-2) avec n = 14 :

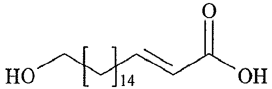

### Caractérisation :

**CCM :** Rf= 0,1 (heptane / acétate d'éthyle 6/4)
**¹H RMN** (300MHz, CDCl₃): δ 1,27-1,65 (m, 26H) ; 2,19-2,39 (m, 2H) ; 3,67 (t, *J=* 6,6 Hz, 2H) ; 5,83 (dt, *J=* 15,6 Hz, 1H) ; 7,09 (dt, *J*= 15,6 Hz, 1H).
**Spectroscopie de Masse :** [M-Na]⁺ 321 (calculé 298)

### Exemple 13 - Réaction de Doebner-Knovenagel à partir de l'oxacyclotridécanol (exemple comparatif)

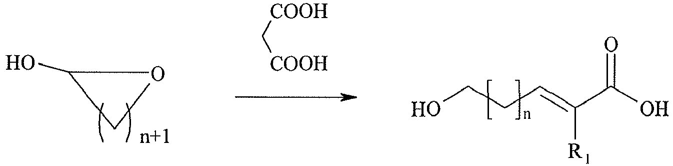

1,4 g (7,0 mmol) d'oxacyclotridécanol obtenu selon le protocole de réduction partielle en lactol (lactol de formule (II) avec n = 13) sont mis en solution, sous flux d'azote, dans 4 volumes de pyridine, en présence de 1,09 g (10,5 mmol) d'acide malonique et 0,11 ml de pipéridine. Le milieu réactionnel est chauffé à 80°C pendant 1 h 30, puis au reflux pendant 2 heures. A température ambiante, la solution est versée sur une solution HCl 3M (50 ml). Le solide filtré est lavé par de l'eau puis est recristallisé dans l'acétonitrile (1,2 g obtenu soit 70% de rendement).

### Exemple 14 - Mode opératoire pour la synthèse de l'acide 18-hydroxy-octadéc-2-fluoro-2-énoïque

Le protocole de synthèse de l'exemple 1 est appliqué au cyclohexadécanolide (Lancaster)(composé de formule (V) avec n = 14) pour conduire à l'acide 18-hydroxy-octadéc-2-fluoro-2-énoïque (rendement = 73%), qui est un composé de formule (I-2) avec n = 14 :

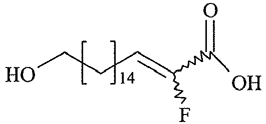

### Caractérisation :

**CCM :** Rf = 0,1 (heptane / acétate d'éthyle 6/4)
**¹H** RMN (300MHz, CDCl₃) : δ 1,28-1,62 (m, 52H) ; 2,28-2,31 (m, 2H, forme *cis) ;* 2.54-2.56 (m, 2H, forme *trans*) ; 3,69 (t, *J =* 6,6 Hz, 4H) ; 6.07 (dt, *J =* 21.6 Hz, 1H, forme *trans*) ; 6.30 (dt, *J =* 33.0 Hz, 1H, forme *cis*).
**Spectroscopie de Masse :** [M-Na]⁺ 339 (calculé 316)

### Exemple 15 - Caractérisation de l'acide 15-hydroxy-pentadéc-2t-énoïque (exemple comparatif)

Le protocole de synthèse de l'exemple 1 est appliqué à un composé de formule (V) avec n = 11 pour conduire à l'acide 15-hydroxy-pentadéc-2*t*-énoïque, qui est un composé de formule (I-2) avec n = 11 :

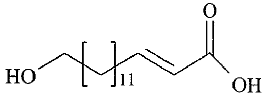

**CCM :** Rf = 0,1 (heptane / acétate d'éthyle 6/4)
**¹H RMN** (300MHz, CDCl₃) : δ 1,28-1,60 (m, 20H) ; 2,21-2,28 (m, 2H) ; 3,66 (t, *J* = 6,6 Hz, 2H) ; 5,83 (dt, *J* = 15,6 Hz, 1H) ; 7,09 (dt, *J* = 15,6 Hz, 1H). **Spectroscopie de Masse :** [M-Na]⁺ 279 (calculé 256)

### Exemple 16 - Caractérisation de l'acide 16-hydroxy-hexadéc-2t-énoïque (exemple comparatif)

Le protocole de synthèse de l'exemple 1 est appliqué à un composé de formule (V) avec n = 12 pour conduire à l'acide 15-hydroxy-hexadéc-2*t*-énoïque, qui est un composé de formule (I-2) avec n = 12 :

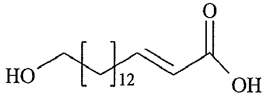

**CCM :** Rf= 0,1 (heptane / acétate d'éthyle 6/4)
**¹H RMN** (300MHz, CDCl₃) : δ 1,28-1,61 (m, 22H) ; 2,21-2,28 (m, 2H) ; 3,66 (t, *J* = 6,6 Hz, 2H) ; 5,85 (dt, *J=* 15,0 Hz, 1H) ; 7,09 (dt, *J* = 15,6 Hz, 1H). **Spectroscopie de Masse :** [M-Na]⁺ 293 (calculé 270)

## Revendications

1. Procédé de préparation d'un composé de formule (I) suivante : 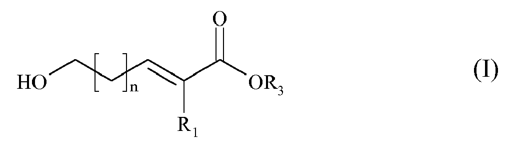 dans laquelle :
• R₃ représente H ou un groupe alkyle R₄, linéaire ou ramifié, comprenant de 1 à 6 atomes de carbone,
• R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
• n est égal à 7, 8, 9, 10, 11, 12, 13 ou 14,
ledit procédé de préparation comprenant :
- la mise en oeuvre de la réaction de Wittig-Horner par la réaction d'un phosphonate de formule (III) suivante : 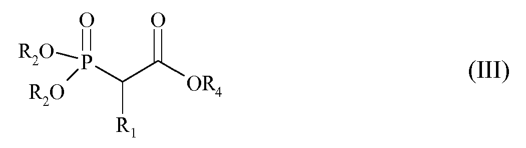 dans laquelle :
- R₁ est tel que défini ci-dessus,
- R₂ représente un groupe alkyle, linéaire ou ramifié, comprenant de 1 à 6 atomes de carbone, et est de préférence un groupe éthyle ou méthyle, lesdits groupes R₂ pouvant former un cycle avec les atomes d'oxygène des groupes OR₂ et l'atome de phosphore du groupe P=O, et
- R₄ représente un groupe alkyle, linéaire ou ramifié, comprenant de 1 à 6 atomes de carbone, et est de préférence un groupe éthyle,
sur un lactol de formule (II) suivante : 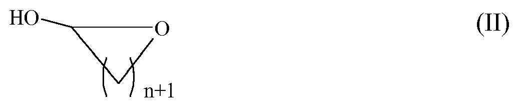 n étant tel que défini ci-dessus,
afin d'obtenir un hydroxy-ester de formule (IV) suivante : 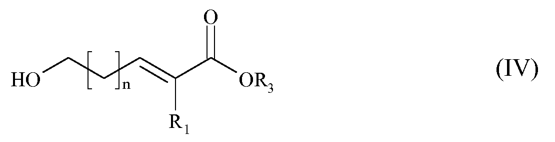 dans laquelle n, R₁ et R₃ sont tels que définis ci-dessus,
- et, éventuellement, lorsque R₃ représente un groupe R₄ tel que défini ci-dessus, une réaction de saponification de l'hydroxy-ester de formule (I) susmentionnée, pour obtenir un hydroxy-acide de formule (I) suivante : 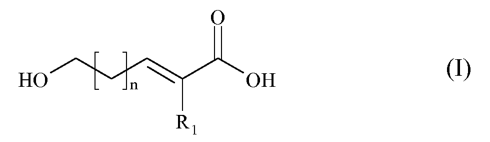
dans laquelle n et R₁ sont tels que définis ci-dessus.

2. Procédé selon la revendication 1, **caractérisé en ce que** le lactol de formule (II) est obtenu par la réduction d'une lactone de formule (V) suivante : 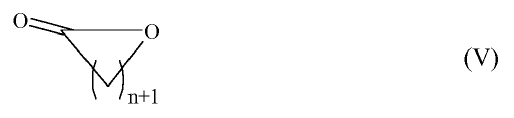 n étant tel que défini dans la revendication 1.

3. Procédé selon la revendication 2, **caractérisé en ce que** la lactone de formule (V) est obtenue à partir d'une cétone de formule (VI) suivante, par la mise en oeuvre de la réaction de Baeyer-Villiger : 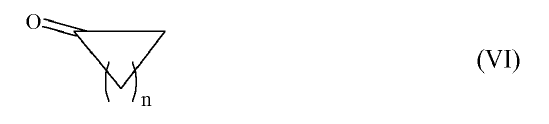 n étant tel que défini dans la revendication 1.

4. Procédé selon la revendication 1, **caractérisé en ce que** le composé de formule (I) est un hydroxy-acide gras insaturé de formule (I-1) suivante : 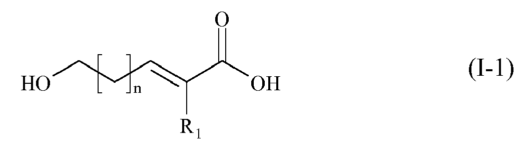 dans laquelle n et R₁ sont tels que définis à la revendication 1,
ledit procédé comprenant en outre l'étape de réduction d'une lactone de formule (V) suivante : 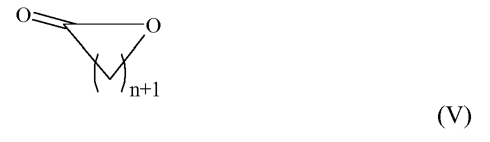 n étant tel que défini ci-dessus,
afin d'obtenir un lactol de formule (II) suivante : 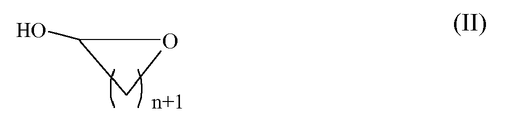 n étant tel que défini ci-dessus.

5. Composé de formule (I) tel qu'obtenu selon le procédé tel que défini selon l'une quelconque des revendications 1 à 4, **caractérisé en ce que** R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène, et n est égal à 7, 8, 9, 10, 11, 12, 13 ou 14.

6. Composé de formule (I-3) suivante : 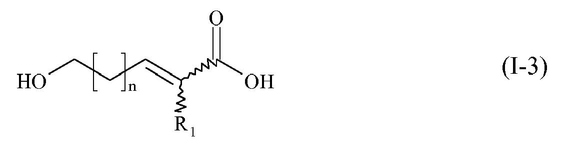 dans laquelle :
• R₁ représente : F, Cl, Br, CF₃ ou un groupe alkyle, linéaire ou ramifié, comprenant de 1 à 6 atomes de carbone, ledit groupe alkyle étant éventuellement substitué par un atome d'halogène ; et
• n est égal à 7, 8, 9, 10, 11, 12, 13 ou 14,
ledit composé étant sous la forme de stéréoisomères Z ou E, ou sous la forme d'un mélange de ces différentes formes.

## Patentansprüche

1. Zubereitungsverfahren einer Verbindung mit der folgenden Formel (I) : 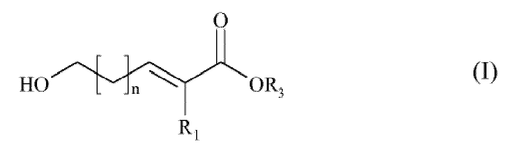 in der:
• R₃ H oder eine lineare oder verzweigte Alkylgruppe R₄ darstellt, umfassend 1 bis 6 Kohlenstoffatome,
• R₁ darstellt: F, Cl, Br, CF₃ oder eine lineare oder verzweigte Alkylgruppe, umfassend 1 bis 6 Kohlenstoffatome, wobei die genannte Alkylgruppe eventuell durch ein Halogenatom substituiert ist; und
• n gleich 7, 8, 9, 10, 11, 12, 13 oder 14 ist, wobei das genannte Zubereitungsverfahren umfasst:
- die Umsetzung der Wittig-Horner-Reaktion durch die Reaktion eines Phosphonats mit der folgenden Formel (III) : 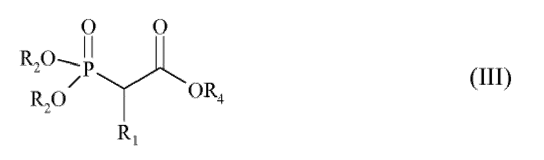 in der:
- R₁ gemäß der obigen Definition ist,
- R₂ eine lineare oder verzweigte Alkylgruppe darstellt, umfassend 1 bis 6 Kohlenstoffatome und bevorzugt eine Ethyl- oder Methylgruppe ist, wobei die genannten Gruppen R₂ einen Zyklus mit den Sauerstoffatomen der Gruppen OR₂ und dem Phosphoratome der Gruppe P=O bilden können und
- R₄ eine lineare oder verzweigte Alkylgruppe darstellt, umfassend 1 bis 6 Kohlenstoffatome und bevorzugt eine Ethylgruppe ist,
auf einem Laktol mit der folgenden Formel (II): 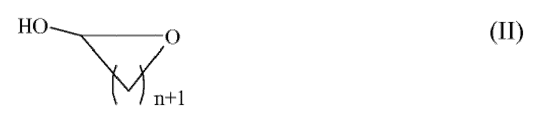 wobei n gemäß der obigen Definition ist,
um einen Hydroxy-Ester mit der folgenden Formel (IV) zu erhalten: 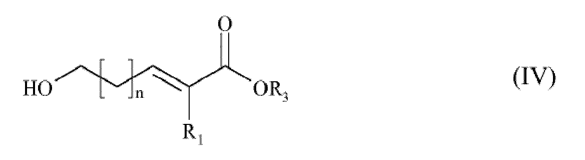 in der n, R₁ und R₃ wie oben definiert sind,
- und eventuell, wenn R₃ eine Gruppe R₄ gemäß obiger Definition darstellt, eine Verseifungsreaktion des Hydroxy-Esters mit der o. g. Formel (I), um ein Säurenhydroxid mit der folgenden Formel (I) zu erhalten: 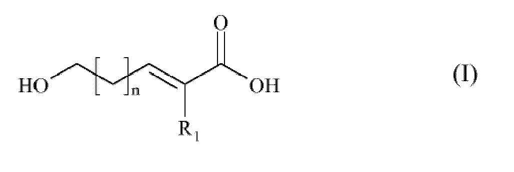
in der n und R₁ gemäß obiger Definition sind.

2. Verfahren gemäß Anspruch 1, **dadurch gekennzeichnet, dass** das Laktol mit der Formel (II) durch die Reduktion eines Laktons mit der folgenden Formel (V) erhalten wird: 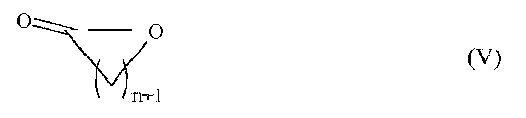 wobei n gemäß der Definition in Anspruch 1 ist.

3. Verfahren gemäß Anspruch 2, **dadurch gekennzeichnet, dass** das Lakton mit der Formel (V) aus einem Ceton mit der folgenden Formel (VI) durch die Umsetzung der Reaktion Baeyer-Viller erhalten wird: 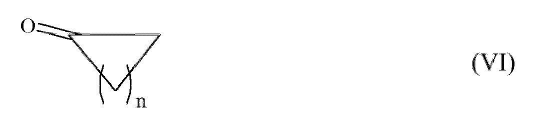 wobei n gemäß der Definition in Anspruch 1 ist.

4. Verfahren gemäß Anspruch 1, **dadurch gekennzeichnet, dass** die Verbindung der Formel (I) ein ungesättigtes Fettsäurenhydroxid mit der folgenden Formel (I-1) ist: 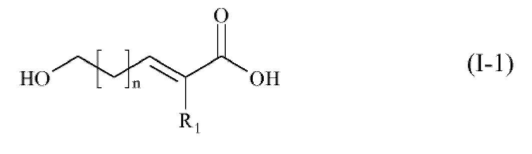 in der n und R₁ gemäß der Definition in Anspruch 1 sind,
wobei das genannte Verfahren darüber hinaus die Reduktionsstufe eines Laktons der folgenden Formel (V) umfasst. 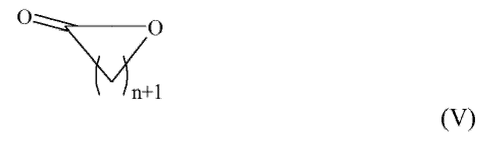 wobei n gemäß obiger Definition ist,
um ein Lakton mit der folgenden Formel (II) zu erhalten: 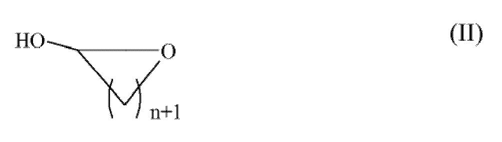 wobei n wie oben definiert ist.

5. Verbindung mit der Formel (I) wie gemäß dem Verfahren erhalten, das gemäß Anspruch 1 bis 4 definiert ist, **dadurch gekennzeichnet, dass** R₁ darstellt: F, Cl, Br, CF₃ oder eine lineare oder verzweigte Alkylgruppe, umfassend 1 bis 6 Kohlenstoffatome, wobei die genannte Alkylgruppe eventuell durch ein Halogenatom substituiert ist und n gleich 7, 8, 9, 10, 11, 12, 13 oder 14 ist.

6. Verbindung mit der folgenden Formel (I-3): 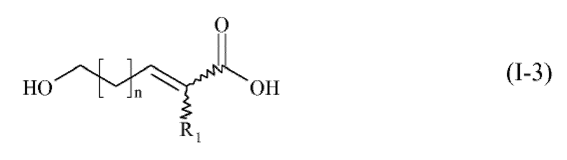 in der:
• R₁ darstellt: F, Cl, Br, CF₃ oder eine lineare oder verzweigte Alkylgruppe, umfassend 1 bis 6 Kohlenstoffatome, wobei die genannte Alkylgruppe eventuell durch ein Halogenatom substituiert ist; und
• n gleich 7, 8, 9, 10, 11, 12, 13 oder 14 ist,
wobei die genannte Verbindung die Form von Stereoisomeren Z oder E oder die Form einer Mischung dieser verschiedenen Formen aufweist.

## Claims

1. A method for preparing a compound of the following formula (I): 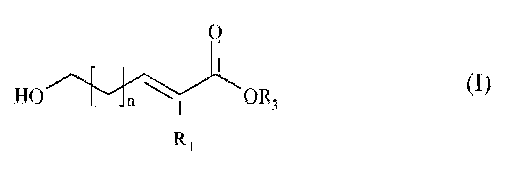 wherein:
• R₃ represents H or a linear or branched R⁴ alkyl group, comprising from 1 to 6 carbon atoms,
• R₁ represents: F, Cl, Br, CF₃, or a linear or branched alkyl group, comprising from 1 to 6 carbon atoms, said alkyl group being optionally substituted by a halogen atom; and
• n is equal to 7, 8, 9, 10, 11, 12, 13, or 14, said preparing method comprising:
- implementing the Wittig-Horner reaction by the reaction of a phosphonate of the following formula (III): 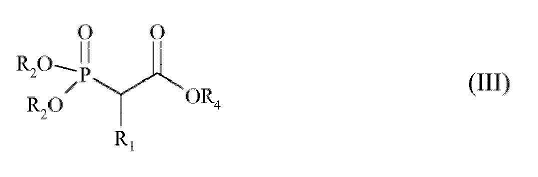 wherein:
- R₁ is as defined above,
- R₂ represents a linear or branched alkyl group, comprising from 1 to 6 carbon atoms, and is preferably an ethyl or methyl group, said R₂ groups being able to form a ring with the oxygen atoms of the OR₂ groups and the phosphorus atom of the P=O group, and
- R₄ represents a linear or branched alkyl group, comprising from 1 to 6 carbon atoms, and is preferably an ethyl group,
on a lactol of the following formula (II): 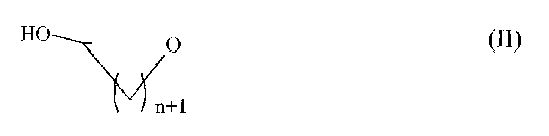 n being as defined above,
in order to obtain a hydroxy-ester of the following formula (IV): 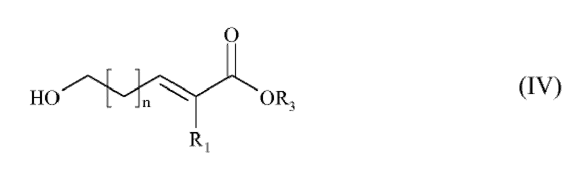 wherein n, R₁ and R₃ are as defined above,
- and, optionally, when R₃ represents an R₄ group as defined above, a saponification reaction of the abovementioned hydroxy-ester of the formula (I), to obtain a hydroxy-acid of the following formula (I): 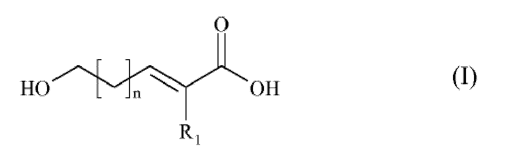
wherein n and R₁ are as defined above.

2. The method according to claim 1, **characterised in that** the lactol of the formula (II) is obtained by reducing a lactone of the following formula (V): 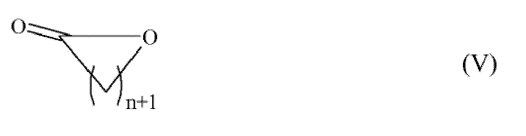 n being as defined in claim 1.

3. The method according to claim 2, **characterised in that** the lactone of the formula (V) is obtained from a ketone of the following formula (VI), by implementing the Baeyer-Villiger reaction: 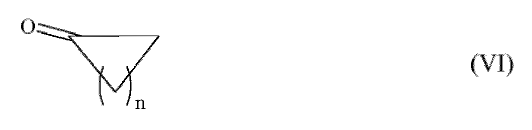 n being as defined in claim 1.

4. The method according to claim 1, **characterised in that** the compound of the formula (I) is a hydroxy-unsaturated fatty acid of the following formula (I-1) : 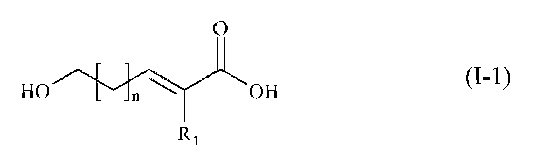 wherein n and R₁ are as defined in claim 1,
said method further comprising the step of reducing a lactone of the following formula (V): 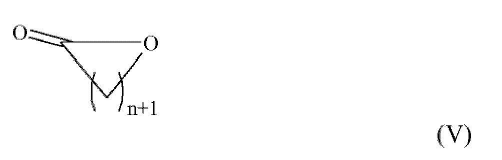 n being as defined above,
in order to obtain a lactol of the following formula (II): 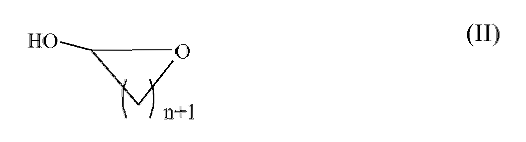 n being as defined above.

5. A compound of the formula (I) as obtained according to the method as defined according to any of claims 1 to 4, **characterised in that** R₁ represents: F, Cl, Br, CF₃, or a linear or branched alkyl group, comprising from 1 to 6 carbon atoms, said alkyl group being optionally substituted by a halogen atom; and n is equal to 7, 8, 9, 10, 11, 12, 13, or 14.

6. A compound of the following formula (I-3) 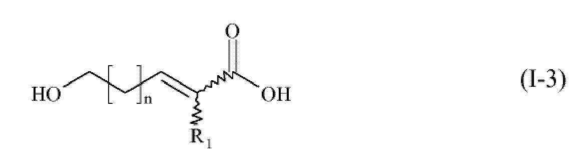 wherein:
R₁ represents: F, Cl, Br, CF₃, or a linear or branched alkyl group, comprising from 1 to 6 carbon atoms, said alkyl group being optionally substituted by a halogen atom; and
n is equal to 7, 8, 9, 10, 11, 12, 13, or 14,
said compound being in the form of Z or E stereoisomers, or in the form of a mixture of these different forms.
